# EUROPÄISCHE PATENTSCHRIFT

(11) **EP 1 210 072 B1**
(45) Veröffentlichungstag und Bekanntmachung des Hinweises auf die Patenterteilung: **31.03.2004**
(21) Anmeldenummer: 00962390.1
(22) Anmeldetag: 26.08.2000
(51) Int. Cl.: A61K 9/70

(54) **KUNSTSTOFFFOLIEN, INSBESONDERE FUR DIE VERWENDUNG IN EINEM DERMALEN ODER TRANSDERMALEN THERAPEUTISCHEN SYSTEM**
PLASTIC FILMS, ESPECIALLY FOR USE IN A DERMAL OR TRANSDERMAL THERAPEUTIC SYSTEM
FEUILLES EN PLASTIQUE, DESTINEES A ETRE UTILISEES EN PARTICULIER DANS UN SYSTEME THERAPEUTIQUE DERMIQUE OU TRANSDERMIQUE

(30) Priorität: 10.09.1999 DE 19943317
(43) Veröffentlichungstag der Anmeldung: 05.06.2002
(73) Patentinhaber: LTS Lohmann Therapie-Systeme AG, 56626 Andernach (DE)
(72) Erfinder: KLEIN, Robert-Peter, 56567 Neuwied (DE); MECONI, Reinhold, 56567 Neuwied (DE); GÖTTE, Ursula, 53572 Unkel (DE)
(74) Vertreter: Flaccus, Rolf-Dieter
(86) Internationale Anmeldenummer: PCT/EP2000/008332
(87) Internationale Veröffentlichungsnummer: WO 2001/019351

(56) Entgegenhaltungen:
- EP-A- 0 186 019
- EP-A- 0 384 267
- WO-A-96/40355
- DE-A- 3 315 272
- US-A- 5 593 684

## Beschreibung

Die Erfindung betrifft beidseitig mit Metallen bedampfte Kunststofffolien, insbesondere für die Verwendung zur Reduzierung der Aufnahme von Wirkstoffen in einem dermalen oder transdermalen therapeutischen System, umfassend ein flächenhaftes, leicht flüchtige Hilfs- und/oder Wirkstoffe enthaltendes Substrat oder Reservoir, welches auf einer seiner Flächen mit einer für die Inhaltsstoffe impermeablen Rückschicht, und auf der Gegenfläche mit einer ablösbaren Schutzschicht zur Verhinderung von Verlust an Inhaltsstoffen bei längerer Lagerung abgedeckt ist.

Transdermale therapeutische Systeme (TTS) umfassen üblicherweise eine für Wirk- oder Hilfsstoff undurchlässige Rückschicht, eine wirkstoffhaltige Schicht und eine ablösbare Schutzschicht. Das Material, aus dem die undurchlässige Rückschicht und die ablösbare Schutzschicht hergestellt werden, ist üblicherweise so ausgewählt, daß es möglichst wenig Wirkstoff aufnimmt.
Bei leicht verdampfenden Wirkstoffen wie z.B. Nitroglycerin und Nikotin gestaltet sich die Auswahl der Materialien jedoch schwierig. Nach dem Stand der Technik verwendet man hierfür Kunststofffolien, die auf der Seite, die mit der wirkstoffhaltigen Schicht in Berührung kommt, einseitig mit Metall bedampft sind. Diese Maßnahme reicht jedoch nicht aus, um Wirkstoffverluste zufriedenstellend zu verhindern, die dadurch entstehen, daß Wirkstoff von den Schnittkanten des transdermalen therapeutischen Systems her entweicht, sich auf der offenen Seite der undurchlässigen Rückschicht und der ablösbaren Schutzschicht niederschlägt und vom Material dieser Schichten aufgenommen wird.

EP 0 186 019 beschreibt in Beispiel 1 ein transdermales therapeutisches System, welches in der wirkstoffhaltigen Schicht Nitroglycerin enthält, das an Lactose adsorbiert ist. Während der Lagerung verliert jedoch die wirkstoffhaltige Schicht Nitroglycerin, und zwar über einen Zeitraum von 12 Monaten ca. 16 %. Das bedeutet, daß das bekannte transdermale therapeutische System instabil ist und pharmazeutischen Anforderungen nicht entspricht.

DE 33 15 272 beschreibt ein transdermales therapeutisches System mit schichtenförmigem Wirkstoffreservoiraufbau. Nach Beispiel 1 wird als Wirkstoff Nitroglycerin verwendet, das an Lactose adsorbiert ist. Als Deckschicht bzw. für die ablösbare Schutzschicht werden Folien aus verschiedenen polymeren Substanzen verwendet, wie z.B. Polyethylenterephthalat, die einseitig mit Aluminium bedampft sein können. Die Ergebnisse aus Stabilitätsversuchen zeigen jedoch, daß im Verlauf von 15 Monaten die Reservoirschicht ca. 11 % Nitroglycerin verliert, wovon sich etwa 6 % in der Deckschicht und etwa 5 % in der ablösbaren Schutzschicht befinden. Ein Wirkstoffverlust von ca. 11 % in 15 Monaten ist aus pharmazeutischer Sicht unakzeptabel.

Der Erfindung liegt die Aufgabe zugrunde, ein dermales oder transdermales therapeutisches System, enthaltend leicht flüchtige Wirk- oder Hilfsstoffe, anzugeben, welches derartig ausgebildet ist, daß unter Überwindung der beim Stand der Technik auftretenden, zu Verlusten an Wirk- und Hilfsstoff führenden Schwierigkeiten und technischen Grenzen selbst bei längerer Lagerung derartige Verluste nahezu vollständig vermieden werden.

Überraschenderweise hat sich gezeigt, daß die Aufgabe durch eine beidseitige Bedampfung von Kunststofffolien mit Metall gemäß Hauptanspruch gelöst wird.

Die auf die Kunststofffolien aufgedampfte Metallmenge kann 10 - 500 mg/m², bevorzugt aber 40 - 200 mg/m² pro Seite betragen, wobei die Kunststofffolien bevorzugt mit Aluminium bedampft werden.

Die beidseitig mit Metall bedampften Kunststofffolien können ein- oder beidseitig abhäsiv ausgerüstet sein. Dies ist vor allem dann erforderlich, wenn die beidseitig mit Metall bedampfte Kunststofffolie als ablösbare Schutzschicht verwendet wird. Zum abhäsiven Ausrüsten der beidseitig mit Metall bedampften Kunstofffolien werden üblicherweise Silikonpolymere verwendet.

Die Kunststofffolien können ausgewählt sein aus der Gruppe von Polyester, Polyethylen, Polypropylen, Polyamid, Polyurethan, Polyvinylchlorid, Polyvinylidenchlorid, Polyvinylalkohol und Ethylenvinylacetat-Copolymeren.

Kunststofffolien können für sich allein zur Bedampfung mit Metallen verwendet werden, sie können jedoch auch miteinander kombiniert sein. Die Kunststofffolie kann also auch ein Laminat aus zwei oder mehreren verschiedenartigen Kunststoffschichten sein.

Üblicherweise weisen die Kunststofffolien eine Dicke von 0,004 - 1,0 mm, bevorzugt 0,010 - 0,5 mm, auf.

Die beidseitig mit Metall bedampften Kunststofffolien, die auch abhäsiv ausgerüstet sein können, finden bevorzugt Verwendung bei der Herstellung von flächenförmigen Arzneimitteln mit leicht flüchtigen Inhaltsstoffen.

Die beidseitige Bedampfung der Kunststofffolien mit Metallen geschieht im Hochvakuum oder mit Unterstützung eines Plasmas und kann in einem oder mehreren Arbeitsgängen durchgeführt werden.

### Beispiel 1

In eine Dünnschichtchromatographie-Glaskammer wurde so viel Nitroglycerin-Lactose-Verreibung (10 % Nitroglycerin) gegeben, daß der Boden gut bedeckt war. In den Dampfraum wurden Folienproben (16 cm²) eingehängt und in einen auf 40 °C erwärmten Trockenschrank gestellt. In Zeitabständen von 1,2 bzw. 3 Monaten wurden Proben entnommen. Diese wurden mit Methanol abgespült, um den adsorbierten Wirkstoff zu entfernen. Anschließend wurden die Prüflinge mit Methanol erschöpfend extrahiert und der Wirkstoff bestimmt.

**Tabelle 1**

| *Folientyp: 100 µm PET* | *Nitroglyceringehalt (µm*/*cm*^{*2*}*) Lagerdauer (Monate)* | | |
|---|---|---|---|
| | 1 | 2 | 3 |
| beidseitig silikonisiert | 0,96 | 0,88 | 1,28 |
| einseitig mit Aluminium bedampft und beidseitig silikonisiert | 0,71 | 0,55 | 0,85 |
| beidseitig mit Aluminium bedampft und beidseitig silikonisiert | 0,05 | 0,12 | 0,11 |

Wie die Ergebnisse (Tab. 1) zeigen, wird von einer beidseitig mit Aluminium bedampften und beidseitig silikonisierten PET-Folie (100 µm) nur etwa 1/10 der Nitroglycerinmenge einer nicht mit Aluminium bedampften Folie aufgenommen (bei 3-monatiger Lagerung bei 40 °C).

### Beispiel 2

Die Versuchsbedingungen waren analog zu denen in Beispiel 1; als Wirkstoff wurde Nikotin verwendet.

**Tabelle 2**

| *Folientyp: 100 µm PET* | *Nikotin (µm*/*cm*^{*2*}*) Lagerdauer* (Monate) | | |
|---|---|---|---|
| | 1 | 2 | 3 |
| beidseitig silikonisiert | 0,24 | 0,59 | 0,75 |
| einseitig mit Aluminium bedampft und beidseitig silikonisiert | 0,22 | 0,41 | 0,68 |
| beidseitig mit Aluminium bedampft und beidseitig silikonisiert | 0,05 | 0 | 0,17 |

Wie die Ergebnisse belegen, ist auch hier durch die beidseitige Bedampfung mit Aluminium die Wirkstoffaufnahme einer 100 µm dicken PET-Folie deutlich reduziert.

## Patentansprüche

1. Dermales oder transdermales therapeutisches System, umfassend ein flächenhaftes, leicht flüchtige Hilfs- oder Wirkstoffe enthaltendes Substrat oder Reservoir, welches auf einer seiner Flächen mit einer für die Inhaltsstoffe impermeablen Rückschicht, und auf der Gegenfläche mit einer ablösbaren, ebenfalls impermeablen Schutzschicht zur Verhinderung von Verlust der Inhaltsstoffe bei längerer Lagerung abgedeckt ist, **dadurch gekennzeichnet,**
**daß** sowohl die Rückschicht, als auch die Schutzschicht aus Kunststofffolie besteht, welche beidseitig mit aufgedampftem Metall impermeabel beschichtet ist.

2. Therapeutisches System nach Anspruch 1, **dadurch gekennzeichnet, daß** die pro Flächeneinheit aufgedampfte Metallmenge bevorzugt 10 bis 500 mg/m², vorzugsweise 40 bis 200 mg/m², beträgt.

3. Therapeutisches System nach einem oder mehreren der Ansprüche 1 bis 2, **dadurch gekennzeichnet, daß** das aufgedampfte Metall Aluminium ist.

4. Therapeutisches System nach einem oder mehreren der Ansprüche 1 bis 3, **dadurch gekennzeichnet, daß** die mit einer Metallschicht bedampften Kunststofffolien der Schutzschicht zumindest einseitig abhäsiv ausgerüstet sind.

5. Therapeutisches System nach einem oder mehreren der Ansprüche 1 bis 4, **dadurch gekennzeichnet, daß** die Kunststofffolien ausgewählt sind aus der Gruppe von Polyester, Polyethylen, Polypropylen, Polyamid, Polyurethan, Polyvinylchlorid, Polyvinylidenchlorid, Polyvinylalkohol und Ethylenvinylacetat-Copolymer.

6. Therapeutisches System nach einem oder mehreren der Ansprüche 1 bis 5, **dadurch gekennzeichnet, daß** die Kunststofffolien eine Dicke zwischen 0,004 und 1,0 mm, vorzugsweise zwischen 0,010 und 0,5 mm, besitzen.

7. Therapeutisches System nach einem oder mehreren der Ansprüche 1 bis 6, **dadurch gekennzeichnet, daß** es als Inhaltsstoff Nitroglyzerin enthält.

8. Therapeutisches System nach einem oder mehreren der Ansprüche 1 bis 6, **dadurch gekennzeichnet, daß** es als Inhaltsstoff Nikotin enthält.

9. Verfahren zur Herstellung von mit einer Metallschicht bedampften Kunststofffolien für transdermale therapeutische Systeme nach einem oder mehreren der Ansprüche 1 bis 8, **dadurch gekennzeichnet, daß** die Bedampfung zur Erzielung absolut impermeabler beidseitiger Metallschichten im Hochvakuum in wenigstens einem Arbeitsgang vorgenommen wird.

10. Verfahren zur Herstellung von mit einer Metallschicht bedampften Kunststofffolien für transdermale therapeutische Systeme nach einem oder mehreren der Ansprüche 1 bis 8, **dadurch gekennzeichnet, daß** die Bedampfung zur Erzielung absolut impermeabler Metallschichten mit Hilfe eines Plasmas in wenigstens einem Arbeitsgang vorgenommen wird.

11. Verwendung von Kunststofffolien nach den Ansprüchen 1 bis 10 zur Herstellung dermaler oder transdermaler therapeutischer Systeme, die leicht flüchtige Wirk- oder Hilfsstoffe enthalten.

## Claims

1. Dermal or transdermal therapeutic system comprising a sheet-like substrate or reservoir containing readily volatile auxiliary agents or active agents, which substrate or reservoir is covered on one of its sides with a backing layer impermeable to the ingredients and on the opposite side with a detachable, likewise impermeable protective layer to prevent loss of ingredients during prolonged storage, **characterized in that**
both the backing layer and the protective layer are made of plastic film which is impermeably coated on both sides with vapour-deposited metal.

2. Therapeutic system according to Claim 1, **characterized in that** the amount of metal which is vapour-deposited per unit area is preferably 10 to 500 mg/m², and more preferably 40 to 200 mg/m².

3. Therapeutic system according to one or more of Claims 1 to 2, **characterized in that** the vapour-deposited metal is aluminium.

4. Therapeutic system according to one or more of Claims 1 to 3, **characterized in that** the plastic films of the protective layer, which are provided with a vapour-deposited metal layer, are rendered abhesive on at least one side.

5. Therapeutic system according to one or more of Claims 1 to 4, **characterized in that** the plastic films are selected from the group of polyester, polyethylene, polypropylene, polyamide, polyurethane, polyvinyl chloride, polyvinylidene chloride, polyvinyl alcohol and ethylenevinyl acetate copolymer.

6. Therapeutic system according to one or more of Claims 1 to 5, **characterized in that** the plastic films have a thickness between 0.004 to 1.0 mm, preferably between 0.010 and 0.5 mm.

7. Therapeutic system according to one or more of claims 1 to 6, **characterized in that** it contains nitroglycerine as ingredient.

8. Therapeutic system according to one or more of Claims 1 to 6, **characterized in that** it contains nicotine as ingredient.

9. Process for the manufacture of plastic films, provided with a vapour-deposited metal layer, for transdermal therapeutic systems according to one or more of claims 1 to 8, **characterized in that** to obtain absolutely impermeable double-face metal layers, the vapour deposition is performed under high vacuum in at least one operation.

10. Process for the manufacture of plastic films, provided with a vapour-deposited metal layer, for transdermal therapeutic systems according to one or more of claims 1 to 8, **characterized in that** to obtain absolutely impermeable metal layers, the vapour deposition is performed with the aid of a plasma in at least one operation.

11. Use of plastic films according to Claims 1 to 10 for manufacturing dermal or transdermal therapeutic systems containing readily volatile active agents or auxiliary agents.

## Revendications

1. Système thérapeutique dermique ou transdermique, comprenant un substrat ou réservoir plan, contenant des adjuvants ou des principes actifs facilement volatils, lequel est recouvert sur l'une de ses faces d'une couche arrière imperméable aux constituants, et sur la surface opposée d'une couche de protection détachable également imperméable destinée à empêcher la perte des constituants en cas de stockage prolongé, **caractérisé en ce que** tant la couche arrière que la couche protectrice sont constituées d'une feuille en plastique recouverte sur ses deux faces de métal métallisé sous vide de manière à l'imperméabiliser.

2. Système thérapeutique selon la revendication 1, **caractérisé en ce que** la quantité de métal métallisé sous vide par unité de surface est de 10 à 500 mg/m², de préférence de 40 à 200 mg/m².

3. Système thérapeutique selon l'une ou plusieurs des revendications 1 à 2, **caractérisé en ce que** le métal métallisé sous vide est de l'aluminium.

4. Système thérapeutique selon l'une ou plusieurs des revendications 1 à 3, **caractérisé en ce que** les feuilles en plastique de la couche protectrice métallisée sous vide avec une couche métallique sont munies - au moins sur une face - d'un apprêt abhésif.

5. Système thérapeutique selon l'une ou plusieurs des revendications 1 à 4, **caractérisé en ce que** les feuilles en plastique sont choisies parmi le groupe du polyester, polyéthylène, polypropylène, polyamide, polyuréthane, chlorure de polyvinyle, chlorure de polyvinylidène, alcool de polyvinyle et copolymères de l'acétate de vinyle éthylène.

6. Système thérapeutique selon l'une ou plusieurs des revendications 1 à 5, **caractérisé en ce que** les feuilles en plastique possèdent une épaisseur comprise entre 0,004 et 1,0 mm, de préférence entre 0,010 et 0,5 mm.

7. Système thérapeutique selon l'une ou plusieurs des revendications 1 à 6, **caractérisé en ce qu'**il contient de la nitroglycérine comme constituant.

8. Système thérapeutique selon l'une ou plusieurs des revendications 1 à 6, **caractérisé en ce qu'**il contient de la nicotine comme constituant.

9. Procédé de production de feuilles en plastique métallisées sous vide avec une couche métallique destinées à des systèmes thérapeutiques transdermiques selon l'une ou plusieurs des revendications 1 à 8, **caractérisé en ce que** la métallisation sous vide est effectuée pour obtenir en au moins une opération sous vide très poussée des couches métalliques absolument imperméables sur leurs deux faces.

10. Procédé de production de feuilles en plastique métallisées sous vide avec une couche métallique destinées à des systèmes thérapeutiques transdermiques selon l'une ou plusieurs des revendications 1 à 8, **caractérisé en ce que** la métallisation sous vide est effectuée en au moins une opération à l'aide d'un plasma pour obtenir des couches métalliques absolument imperméables.

11. Utilisation de feuilles en plastique selon les revendications 1 à 10 pour la production de systèmes thérapeutiques dermiques ou transdermiques qui contiennent des principes actifs ou des adjuvants facilement volatils.
